# EUROPEAN PATENT SPECIFICATION

(11) **EP 1 980 571 B1**
(45) Date of publication and mention of the grant of the patent: **13.10.2010**
(21) Application number: 07290440.2
(22) Date of filing: 11.04.2007
(51) Int. Cl.: C07K 14/47, A61K 38/17

(54) **Anti-tumor drug, medicament, composition, and use thereof**
Antitumorheilmittel, Medikament, Zusammensetzung und Verwendung davon
Médicament anti-tumoral, médicament, composition et leurs utilisations

(43) Date of publication of application: 15.10.2008
(73) Proprietor: GENE SIGNAL INTERNATIONAL SA, Epalinges, VD (CH)
(72) Inventor: Colin, Sylvie, 75014 Paris (FR); Al-Mahmood, Salman, 75014 Paris (FR)
(74) Representative: Icosa

(56) References cited:
- WO-A-02/18440
- WO-A-03/074073
- WO-A-2004/032842
- WO-A-2005/079766
- US-A1- 2005 176 044
- DATABASE NCBI [Online] Hypothetical protein 1 March 2001 (2001-03-01), OSADA N ET AL.: "Direct submission" XP002450545 retrieved from SWISSPROT accession no. Q9GMU7 Database accession no. Q9GMU7
- DATABASE NCBI [Online] Transmembrane 4 superfamily member 2 1 October 2003 (2003-10-01), XP002450546 retrieved from SWISSPROT accession no. Q7YQH1 Database accession no. Q7YQH1 -& KITANO T ET AL.: "Gene Diversity Patterns at 10 X-Chromosomal Loci in Humans and Chimpanzees" MOLECULAR BIOLOGY AND EVOLUTION, vol. 20, no. 8, 2003, pages 1281-1289, XP002450538

## Description

The present invention relates to the field of treatments for cancers. More specifically, the present invention relates to the treatment of cancers by polypeptides derived from a protein belonging to the tetraspanin super family.

Cancer is a class of diseases or disorders characterized by uncontrolled division of cells and the ability of these cells to spread, either by direct growth into adjacent tissue through invasion, or by implantation into distant sites by metastasis (where cancer cells are transported through the bloodstream or lymphatic system). Cancer may affect people at all ages, but risk tends to increase with age. It is one of the principal causes of death in developed countries.

There are many types of cancer. Severity of symptoms depends on the site and character of the malignancy and wether there is metastasis. Once diagnosed, cancer is usually treated with a combination of surgery, chemotherapy and radiotherapy. As research develops, treatments are becoming more specific for the type of cancer pathology. Drugs that target specific cancers already exist for several cancers. If untreated, cancers may eventually cause illness and death, though this is not always the case.

Current treatments target distinct properties of malignant cells, such as for example evading apoptosis, unlimited growth potential (immortalization) due to overabundance of telomerase, self-sufficiency of growth factors, insensitivity to anti-growth factors, increased cell division rate, altered ability to differentiate, no ability for contact inhibition, ability to invade neighbouring tissues, ability to build metastases at distant sites, ability to promote blood vessel growth (angiogenesis).

Tumor angiogenesis is the proliferation of a network of blood vessels that penetrates into the tumor, supplying nutrients and oxygen and removing waste products. Tumor angiogenesis actually starts with cancerous tumor cells releasing molecules that send signals to surrounding normal host tissue. This signalling activates certain genes in the host tissue that, in turn, make proteins to encourage growth of new blood vessels. Solid tumors must stimulate the formation of new blood vessels in order to obtain the nutrients and oxygen necessary for their growth, thus providing a route by which the tumors can metastasize to distant sites.

Experimental evidence has suggested that malignant tumors can induce angiogenesis through the elaboration of a variety of factors, such as acidic fibroblast growth factor (aFGF), basic fibroblast growth factor (bFGF), vascular endothelial growth factor (VEGF), platelet derived growth factor (PDGF), transforming growth factor alpha (TGF-alpha), tumor necrosis growth factor alpha (TNF-alpha), and many others (Liotta et al., 1991, Cell 64: 327-336*;* Hanahan et al., Cell 86: 353-364).

Nowadays, plenty of chemotherapeutic molecules targeting angiogenesis are available on the market. Well known naturally occurring angiogenesis inhibitors are angiostatin, endostatin, interferons, platelet factor 4, prolactin 16Kd fragment, thrombospondin, TIMP-1 (tissue inhibitor of metalloprotease -1), TIMP-2 and TIMP-3. These molecules can be used as chemotherapeutic treatments, as well as other drugs such as for example combrestatin A4, EMD 121974, TNP-470, squalamine, thalidomide, interferon-alpha, anti-VEGF, antibodies... However, their efficiency is never sufficient and alternative treatments are desirable.

There is therefore a need of alternative chemotherapeutic agents for the treatment of tumors, having increased efficiency, being less invasive or toxic, and resulting in an increased rate of recovery.

WO 03/074073, in the name of the Applicant, describes a family of 54 genes involved in the regulation of angiogenesis. Amongst these genes, "gene 497" (SEQ ID N°1 in this specification), which encodes "protein 497" (SEQ ID N°2 in this specification), also called TM4SF2 in WO 03/074073, has been described as implied in the activation of angiogenesis (pro-angiogenic). The expression of gene 497 is enhanced when angiogenesis is stimulated by pro-angiogenic factors such as VEGF and FGF2. WO 03/074073 also describes that the expression of an antisens of the gene 497, i.e. the inhibition of the expression of the gene 497, in human endothelial cells inhibits the formation of capillary tubes.

Bio-informatics analysis revealed that protein 497C, which comprises 244 amino-acids, contains one extracellular loop SEL (Small Extracellular Loop), one extracellular loop LEL (Large Extracellular Loop), four trans-membrane spans and two intracellular tails corresponding to the N- and C-terminals. Thus, this protein has been classified as a member of the tetraspanin super family (Levy et al., Nat Rev Immunol. 2005 Feb;5(2):136-48).

The tetraspanins are a large family of evolutionarily conserved cell-surface proteins that are expressed in a wide range of organisms. Members of this family tend to associate with each other, together with their partners, in membrane microdomains that provide a scaffold for the transmission of external stimuli to intracellular-signalling components. Basically, tetraspanins comprise four transmembrane (TM) domains which contain conserved polar residues and flank the small and large extracellular loops (SEL and LEL respectively). The LEL is composed of a core formed by helices a, b and e, and this core structure is conserved among the tetraspanins. Helices c and d comprise the variable portion of the LEL, and they are flanked by the CCG motif and further conserved cysteine residues. This region is folded as a result of disulphide bridges to form a mushroom-like structure.

The members of this super-family have been classified into three groups based on the number of cysteine residues present in the LEL domain. Group 1 contains four cysteines in the LEL domain, group 2 contains six cysteines in the LEL domain, and group 3 contains eight cysteines in the LEL domain.

Since protein 497C contains six cysteines within its LEL domain, it could therefore be classified in group 2, similarly to protein CD151, another member of this family. The LEL domain of these proteins comprises 6 domains: a, b, c, d1, d2 and e, among which d1, d2 and c consist in the variable portions of the LEL.

Going deeper in their researches, the inventors produced truncated forms of protein 497C, corresponding to the various fragments of the extracellular domain SEL and LEL of protein 497C:
- 497C-T2: entire LEL domain of protein 497C, identified by SEQ ID NO :3 in this specification (112 amino acids),
- 497C-T3: c, d1, d2 and e domains of the LEL domain, identified by SEQ ID NO :4 in this specification (74 amino acids),
- 497C-T4: d1, d2 and e domains of the LEL domain, identified by SEQ ID NO :5 in this specification (49 amino acids),
- 497C-T5: d2 and e domains of the LEL domain, identified by SEQ ID NO :6 in this specification (43 amino acids).

To ensure the three-dimensional configuration of these fragments, i.e. the three-dimensional conformation of the LEL domain, and therefore their potential activity inventors added, according to an embodiment, a tail at the C-terminus of the fragments. This tail was composed of a random sequence of 30 to 70 amino acids, preferably of 45 to 65 amino acids, more preferably of 50 to 60 amino acids, still more preferably of about 55 amino acids. This tail has no activity *per se*, and its presumed role is to stabilize the three-dimensional structure of the LEL fragment, i.e. to maintain the polypeptide folded in a biologically active form.

In a first experiment, the inventors found that protein 497C-T2 may inhibit human endothelial cell proliferation *in vitro* in a dose dependent manner.

Then, in a second experiment, the inventors surprisingly found that the truncated forms of protein 497C may have a strong activity to inhibit capillary tube formation *in vitro,* in a dose dependent manner.

Other experiments conducted by the inventors suggested that truncated forms of protein 497C induced the inhibition of the migration of endothelial cells *in vitro,* in a dose dependent manner. The results of the dose-response study on the inhibition of angiogenesis by 497C-T2 revealed that at 270 nM, the recombinant protein 497C-T2 induced a small inhibition while at 540 nM, 497C-T2 may inhibit by more than 50% *in vitro* angiogenesis (i.e. IC₅₀< 540 nM). This demonstrated that the recombinant protein 497C-T2 is a potent anti-angiogenic compound, and that it could be at least 200-fold more potent than the anti-VEGF mAb and/or VEGF receptor (KDR)-based identified peptides (Binetruy-Tournaire R et al., Identification of a peptide blocking vascular endothelial growth factor (VEGF)-mediated angiogenesis, EMBO J. 2000; 19: 1525-1533).

Capillary tube formation, human endothelial cell proliferation and human endothelial cell migration are three essential steps of angiogenesis. Consequently, the fact that 497C-T2 may inhibit *in vitro* capillary tube formation, human endothelial cell proliferation and/or migration in a dose-dependant manner, thus constituted a strong evidence of the potent anti-angiogenic activity of the truncated forms of protein 497C.

All these results were absolutely unexpected since native protein 497C has been disclosed as pro-angiogenic and that the expression of an antisens of the gene 497, i.e. the inhibition of gene 497, in human endothelial cells inhibits the formation of capillary tubes. It was therefore really surprising that truncated forms of protein 497C, may have anti-angiogenic activity.

In addition, inventors tested the effect of 497C-T2 on the proliferation of many cell lines, including the fibroblast cell line MRC5, CHO, the tumor cell lines Calu-6, NCI-H460, and more, without any detectable antiproliferative effect even at high concentration. In contrast, 497C-T2 significantly inhibited human endothelial cell proliferation for concentration as low as 500 nM, reaching 75% inhibition at 5 µM. Hence, in addition of being more potent than the anti-VEGF therapeutic strategies available today, 497C-T2 is highly specific to endothelial cells.

Interestingly, the inventors found that the truncated form 497C-T3, which lack the a and b domains, was almost as potent than 497C-T2 on the inhibition of angiogenesis and cell migration, indicating that the a and b domains only slightly contribute to the biological activity and/or to the three dimensional conformation necessary for the biological activity of 497C-T2. The truncated forms 497C-T4 (lacking the a, b, and c domains), and 497C-T5 (lacking the a, b, c, and d1 domains) had limited activities relative to that of 497C-T2, indicating that both the c and d1 domains are necessary for the activity of the protein 497C-T2.

These results were also unexpected, and led the inventors to test the anti-tumor activity of 497C-T2 in mice *in vivo*.

Still surprisingly, the inventors found that protein 497C-T2 had a strong anti-tumor activity *in vivo,* and a strong synergistic activity in combination with other chemotherapeutic agent such as for example cisplatin.

Inventors found that the test substance 497C-T2 was not toxic in Nude mice bearing human NCI H460 or CALU-6 tumors at different tested doses. Moreover, 497C-T2 exhibited a strong statistically significant anti-tumoral activity against NCI H460 and CALU-6 tumors as early as two days after the beginning of the treatment (2^{nd} IP injection combined with CDDP). This anti-tumoral activity was persistent during the treatment period. The anti-tumoral effect of 497C-T2 in this model of human lung cancer represented a realistic therapeutic approach as a monotherapy. Its efficacy was strongly potentiated when combined with the cytotoxic anticancer drug CDDP (Cisplatin), which suppressed tumor growth. Cisplatin alone, on the other hand, did not eradicate tumor growth.

It is not clear whether the anti-angiogenic activity of 497C-T2 is solely responsible for its anti-tumoral activity. It is now accepted that biological compounds such as interferon, EGF and Her-2 receptor antagonists can modulate host responses and enhance the efficacy of standard chemotherapies. The data strongly suggested that 497C-T2 may be of use either as a primary anti-tumoral agent or as an add-on synergic therapy to primary cytotoxic agents for the treatment of cancers.

As mentioned above, it is now established that protein 497C is expressed in endothelial cells, and that its expression is enhanced when angiogenesis is stimulated by pro-angiogenic factors such as VEGF and FGF2 (see WO 2003/074073). Protein 497C belongs to the Tetraspanins superfamily, which are transmembrane receptors comprising two extracellular loops, implied in the recognition of extracellular signals, and two intracellular tails (C-terminal and N-terminal), implied in the transduction of the signal. A key feature in the signal transduction is the formation of a ligand-receptor complex between the extracellular loop of the receptor and the ligand. Without wanting to be bound with a theory, Applicants think that the truncated forms of 497C may play their role through a "soluble receptor mechanism": truncated forms of 497C may remain soluble on the surface of the cell and may be recognized by the ligand. As a result, there may be a competition in the recognition of the ligand between the soluble forms of 497C (the fragments of the invention) and the native transmembrane protein, and consequently a decrease, in a dose dependent manner, of the transduction of the pro-angiogenic signal, therefore resulting in the inhibition of angiogenesis and then in the decrease of tumour volume.

The present invention thus relates in a first aspect, to an active polypeptide consisting of the amino acid sequence of SEQ ID NO: 3.

As used herein, "polypeptide" means molecules formed from the linking, in a defined order, of at least 100 amino acids.

As used herein, "active polypeptide" means polypeptides which have a biological activity. In the present invention the polypeptides have an anti angiogenic and anti tumour activity.

In an embodiment, the polypeptides according to the invention further comprise means for having it folded in an active three dimensional conformation. Preferably, said means for having it folded in an active conformation consists in any sequence comprising from 30 to 70 amino acids, preferably from 45 to 65 amino acids, more preferably from 50 to 60 amino acids, still more preferably of about 55 amino acids, said sequence being fused to the C-terminus of said polypeptide.

As used herein, "three dimensional conformation" means the tertiary structure of a polypeptide, i.e. its overall shape, in which the polypeptide performs a biological function.

In a second aspect, the present invention relates to a medicament comprising a polypeptide, as described above.

In a third aspect, the present invention relates to a pharmaceutical composition comprising a polypeptide as described above, and one or more pharmaceutically-acceptable excipients.

In a fourth aspect, the invention relates to a pharmaceutical composition comprising a polypeptide as described above, and one or more pharmaceutically-acceptable excipients, for use in a method of treatment of cancer and/or tumors of the human or animal body.

In a particular embodiment, the pharmaceutical compositions as described above further comprise at least one another active substance selected from anti-angiogenic substances or anti-tumor substances. These substances may be chosen by the man in the art, regarding the effect to be achieved. Preferably, these substances can be selected from cisplatin, carboplatin, etoposide, ifosfamide, mitomycin, vinblastine, vinorelbine, gemcitabine, paclitaxel, docetaxel, and irinotecan.

In a fifth aspect, the invention relates to a pharmaceutical composition comprising synergistic effective amounts of
- a polypeptide as described above, and
- a platinum complex selected from the group consisting of cisplatin and carboplatin.

The medicament or composition useful in the practice of this invention is administered to the mammal by known conventional routes. The medicament or composition described herein may be administered by the same route, or by different routes. For example, the medicament or composition may be administered to patients orally or parenterally (intravenously, subcutaneously, intramuscularly, intraspinally, intraperitoneally, and the like).

When administered parenterally, the composition is preferably formulated in a unit dosage injectable form (solution, suspension, emulsion) with at least one pharmaceutically acceptable excipient. Such excipients are typically nontoxic and non-therapeutic. Examples of such excipients are water, aqueous vehicles such as saline, Ringer's solution, dextrose solution, and Hank's solution and non-aqueous vehicles such as fixed oils (e.g., corn, cottonseed, peanut and sesame), ethyl oleate, and isopropyl myristate. Sterile saline is a preferred excipient. The excipient may contain minor amounts of additives such as substances that enhance solubility, isotonicity, and chemical stability, e.g., antioxidants, buffers, and preservatives. When administered orally (or rectally) the compounds will usually be formulated into a unit dosage form such as a table, capsule, suppository, or cachet. Such formulations typically include a solid, semi-solid or liquid carrier or diluent. Exemplary diluents and excipients are lactose, dextrose, sucrose, sorbitol, mannitol, starches, gum acacia, calcium phosphate, mineral oil, cocoa butter, oil of theobroma, alginates, tragacanth, gelatin, methylcellulose, polyoxyethylene, sorbitan monolaurate, methyl hydroxybenzoate, propyl hydroxybenzoate, talc and magnesium stearate. In preferred embodiments, the pharmaceutical composition according to the invention is administered intravenously.

According to the invention, the amount of polypeptide present in the medicament or composition is effective to treat susceptible tumors. Preferably, the polypeptide is present in an amount from 0.01 to 90% in weight, preferably from 0.1% to 10% in weight, more preferably from 1% to 5% in weight, in the medicament or in the composition. These amounts are routinely adaptable by the man in the art, who is able to choose the best quantity to administer to a patient to achieve recovery.

In a sixth aspect, the invention relates to the use of the polypeptide as described above, or of the medicament as described above, or of the pharmaceutical composition as described above, for the treatment of cancers and/or tumors.

According to the invention, the tumors to be treated are preferably solid tumors. More preferably, the tumors to be treated are selected from sarcomas, carcinomas, and lymphomas. Examples of such tumors are bladder cancer, melanoma, breast cancer, non-Hodgkin's lymphoma, brain cancer, bone cancer, colon and rectal cancer, liver cancer, pancreatic cancer, endometrial cancer, prostate cancer, kidney cancer, skin cancer (non-melanoma), thyroid cancer, lung cancer (small cell lung cancer and non small cell lung cancer).

The present invention further describes a method of treatment comprising administering to a subject in need of treatment the polypeptide as described above, or the medicament as described above, or the pharmaceutical composition as described above, in an amount sufficient to inhibit cancer or tumor growth.

Particularly, the invention describes the method of treatment as described above further comprising administering at least one other anti-neoplastic or anti-tumor drug.

In these methods, administering comprises topical administration, oral administration, intravenous administration, or intraperitoneal administration.

The present invention further describes a method of treatment comprising administering to a subject in need of treatment a synergistic effective amount of
- a polypeptide as described above, and
- a platinum complex selected from the group consisting of cisplatin and carboplatin,
which is sufficient to inhibit cancer or tumor growth.

In one embodiment, said polypeptide and said platinum complex, are administered simultaneously.

In another embodiment, said polypeptide and said platinum complex are administered sequentially. Preferably, said polypeptide or fragments thereof and said platinum complex are administered by separate routes, i.e. orally or parenterally (intravenously, subcutaneously, intramuscularly, intraspinally, intraperitoneally, and the like).

In a particular embodiment, said platinum complex is cisplatin.

In another particular embodiment said platinum complex is carboplatin.

The present invention will now be further described with reference to the following non-limiting examples.
Figure 1 is a diagram representing the % inhibition of endothelial cell proliferation *in vitro* with increasing concentrations of protein 497C-T2.
Figures 2a, 2b, 2c, 2d, 2f, 2g, 2h and 2i are pictures of *in vitro* angiogenesis of endothelial cells in different conditions:

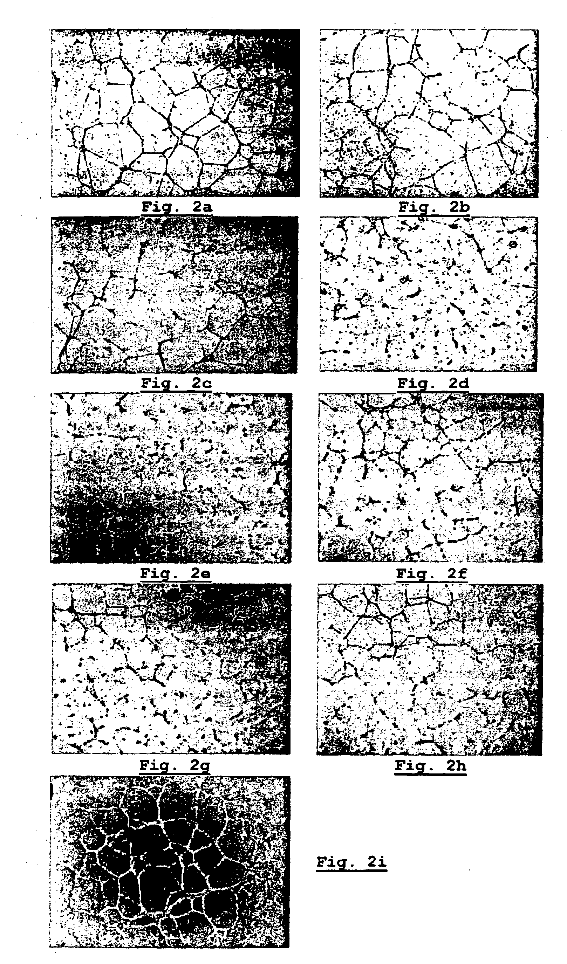

   - Figure 2a: Control (Buffer Urea 2M)
   - Figure 2b: 497C-T2 6.5µg/mL (0.27µM)
   - Figure 2c: 497C-T2 13µg/mL (0.54µM)
   - Figure 2d: 497C-T2 26µg/mL (1.08µM)
   - Figure 2e: 497C-T2 48µg/mL (2µM)
   - Figure 2f: 497C-T3 51µg/mL (2.55µM)
   - Figure 2g: 497C-T4 55µg/mL (3.18µM)
   - Figure 2h: 497C-T5 56µg/mL (3.35µM)
   - Figure 2i: C-terminal tail 53µg/mL (4.14µM)

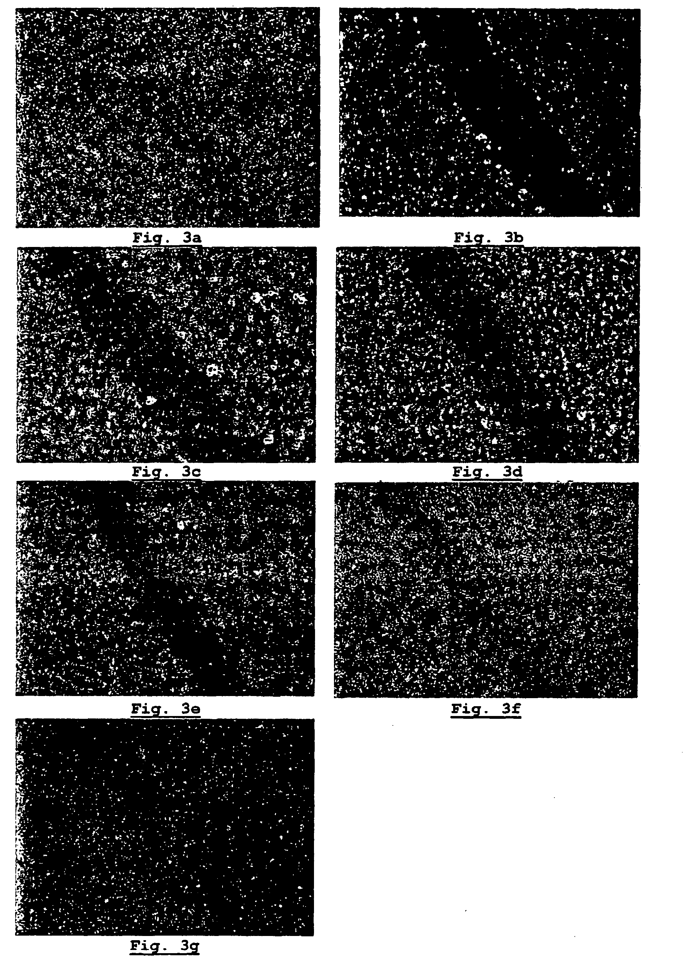

Figures 3a, 3b, 3c, 3d, 3e, 3f, 3g are pictures of wound assay on endothelial cells performed in different conditions:
   - Figure 3b: 497C-T2 20µg/mL (0.83µM)
   - Figure 3c: 497C-T2 40µg/mL (1.66µM)
   - Figure 3d: 497C-T3 48µg/mL (2.4µM)
   - Figure 3e: 497C-T4 60µg/mL (3.52µM)
   - Figure 3f: 497C-T5 61µg/mL (3.65µM)
   - Figure 3g: C-terminal tail 63µg/mL (4.92µM)

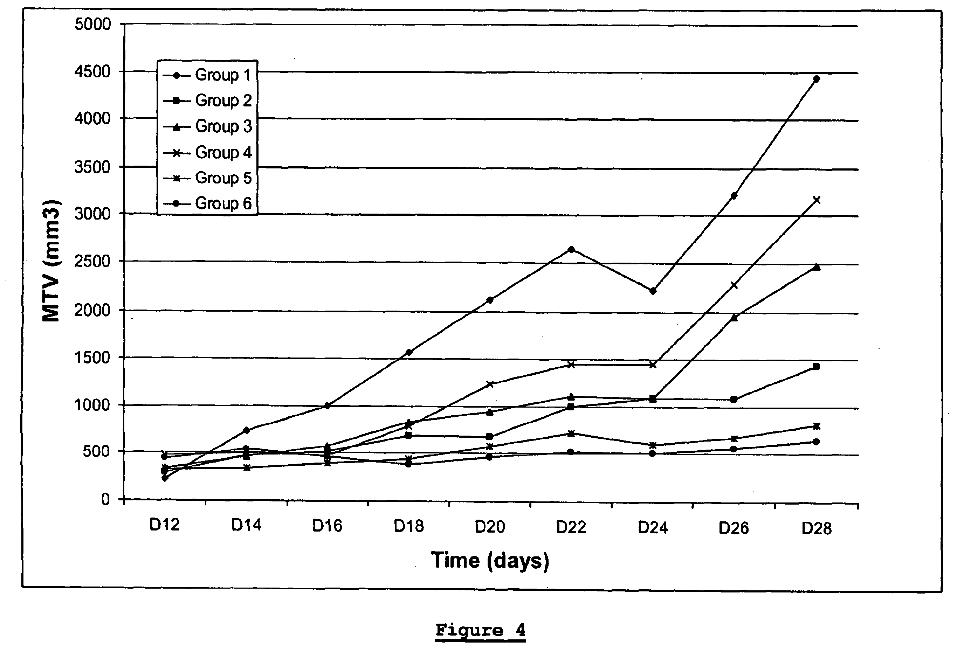

Figure 4 is a graph representing Mean Tumor Volume (mm³) versus time (days) for different groups of mice treated according to example 7.

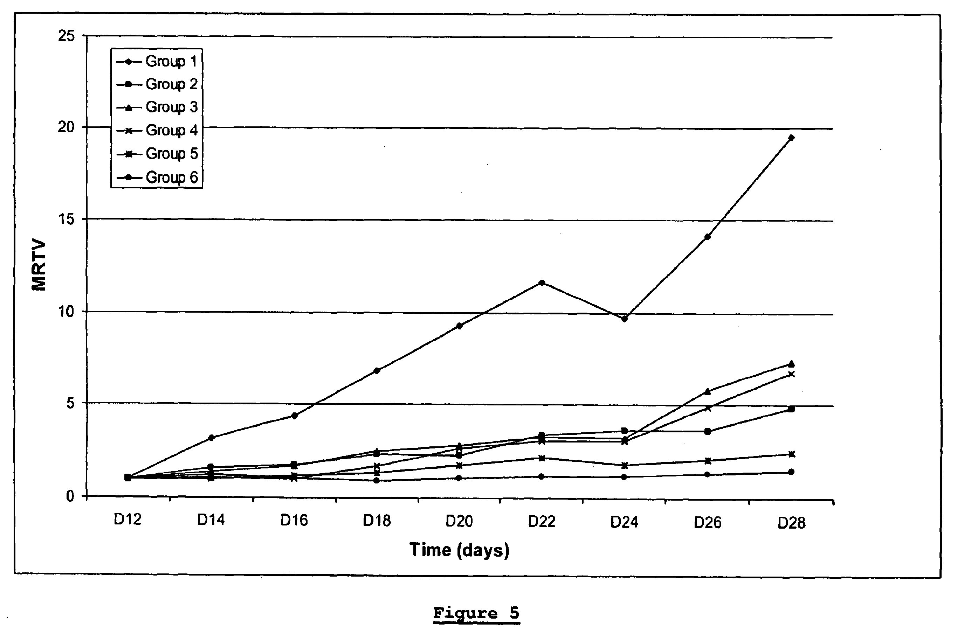

Figure 5 is a graph representing Mean Relative Tumor Volume (without unit) versus time (days) for different groups of mice treated according to example 7.

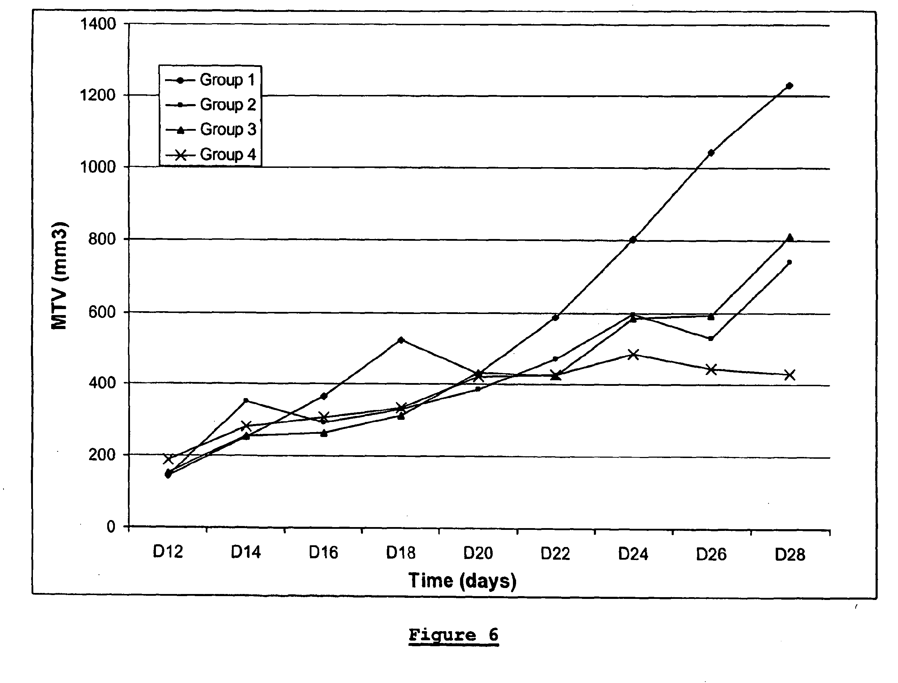

Figure 6 is a graph representing Mean Tumor Volume (mm³) versus time (days) for different groups of mice treated according to example 8.

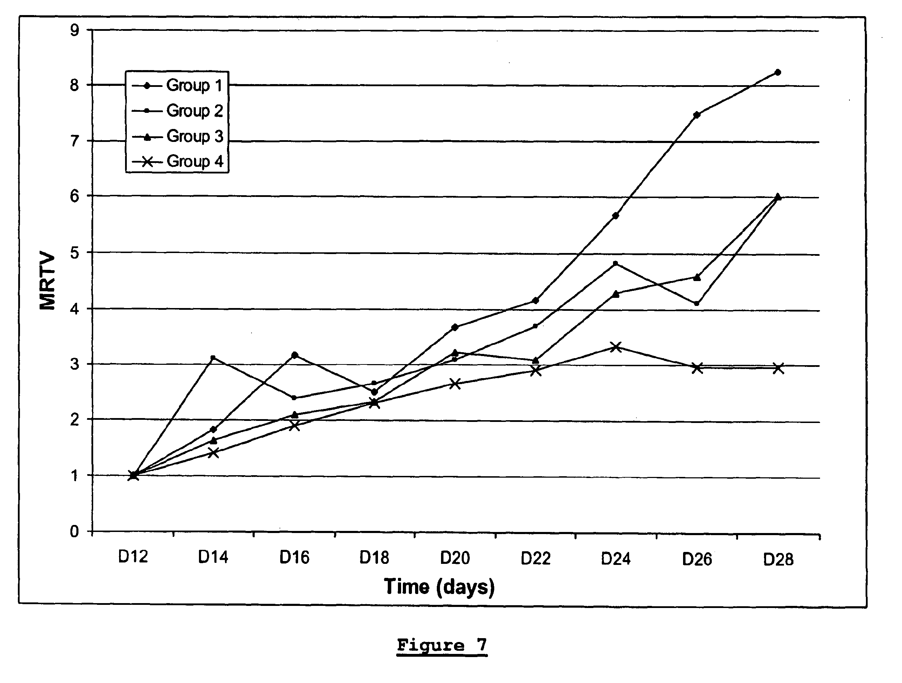

Figure 7 is a graph representing Mean Relative Tumor Volume (without unit) versus time (days) for different groups of mice treated according to example 8.

### EXAMPLE 1: Production of protein 497C-T2

*Synthesis of insert 497C-T2:*

First, gene 497C was cloned in pGEM®-T easy vector system (Promega®) according to known procedures (the vector obtained was called "pGEM-T-497C").

Second, the insert T2 (SEQ ID NO:3) corresponding to the extra-cellular part LEL of the protein 497C was amplified by PCR using the plasmid "pGEM-T-497C" and the two primers CDS5 (SEQ ID NO:9) and CDS4 (SEQ ID NO:10) (table 1), which frame the LEL domain in 5' and 3',respectively.

**Table 1**

| Primer | SEQ ID NO | Sequence |
|---|---|---|
| 497c-cds-5 | 9 | GACGACGACAAGATttcagggtttgtgtttcgtcatgagatcaa |
| 497c-cds-4 | 10 | *GAGGAGAAGCCCGGT*ctccagcgatgattcccatgtt |

Third, the DNA sequence coding for the protein 497C-T2 was inserted into the vector pET-30 EK/LIC (Novagen®) according to known procedures (pET-30-497C-T2). The nucleic acid sequence coding for 497C-T2 within the pET-30 vector is given in SEQ ID NO:7.

The purified vector was then introduced in *E. coli* BL21(DE3) pLys for protein production. Colonies were controlled for the presence of both the vector end the insert by PCR.

The size of the produced protein 497C-T2 was 24 kD, and it was higher than the expected size. This was due to the addition of a supplementary tail at the C-terminal (54 random amino acids) as well as of a His-Tag at the N-terminal as confirmed by sequencing. The amino acid sequence of the protein 497C-T2 as produced is given in SEQ ID NO:8. The protein 497C-T2 was produced within the insoluble fraction of the bacteria, which necessitated an extraction in denaturating conditions.

### Extraction and purification of the protein 497C-T2

Following culture, bacteria were lyzed, centrifuged and the supernatant discarded. The insoluble fraction obtained was treated with Tris-HCl 20 mM, urea 8 M, imidazol 5 mM, NaCl 0.5 M, GSH 5 mM, pH 8.0. After this treatment, the suspension was centrifuged and the supernatant collected, filtered on 0.45µm membranes to discard insoluble materials. The filtered extract was then used to purify the protein 497C-T2 by using a His-Trap column (Amersham®) connected to a HPLC system (Amersham).

The purified protein obtained was diluted in 4 M urea and 0.3 M imidazol. To remove these agents from the preparation, the solution was subjected to dialysis at 4°C.

Following these steps of dialysis, the purified protein was centrifuged at 4,000 x g for 15 min and filtered on 0.45 µm membranes to eliminate possible precipitates. The purified protein preparation was controlled for protein content according to the method described by Bradford in 1976 (Anal. Biochem. 72:248-54) and by SDS-PAGE. The gels were analyzed using the Gene Genius software to quantify the purity by image analysis.

To increase purity of the protein 497C-T2, we performed a second purification step by using ion exchange liquid chromatography. The HisTrap purified preparation was diluted 3 times with the buffer Tris-HCl 20 mM, pH 8, 2 M urea (to decrease the concentration of NaCl to 50 mM), and loaded on MonoS column connected to a HPLC system run by Unicorn software (Amersham, GE, Saclay, France). The column was then washed extensively and eluted with a linear gradient of ionic force (0.05 M to 0.5 M NaCl in the Tris-HCl 20 mM buffer, pH 8, 2 M urea). The purified protein preparation was controlled for protein content both by Bradford and by SDS-PAGE.

**EXAMPLE 2**: Design and production of the truncated forms 497C-T3, 497C-T4, and 497C-T5.

To identify the active site of the protein 497C-T2, three others truncated forms called 497C-T3, 497C-T4, and 497C-T5 were designed and produced as well as a control polypeptide corresponding to both the N-terminal attached His-Tag and the C-terminal tail coming from the vector. To do so, 5' specific primers were designed allowing the progressive truncation of the N-terminal sequence of the protein 497C-T2 without affecting the N-terminal His-Tag necessary for the purification of the produced truncated forms.

### 497C-T3

The truncated 497C-T3 was built by PCR amplification of the DNA using the vector pET30-497C-T2 as template, the 5' specific primer 497c-cds7 (SEQ ID NO:11), which permitted to eliminate most of the b domain and to preserve the first C-C bridge, and the 3' specific primer 497C-cds4(SEQ ID NO:10).

**Table 2**

| Primer | SEQ ID NO | Sequence |
|---|---|---|
| 497c-cds7 | 11 | 5'GACGACGACAAGATGCAGCGCAGCCTGAGCTGC 3' |
| 497c-cds-4 | 10 | 3'GAGGAGAAGCCCGGTctccagcgatgattcccatgtt 5' |

### 497C-T4

The truncated 497C-T4 was built by PCR amplification of the DNA using the vector pET30-497C-T2 as template, the 5' specific primer 497c-cds9 (SEQ ID NO:12), which permitted to eliminate the first C-C bridge, the b and c domains, and to preserve the d1 domain, and the 3' specific primer 497C-cds4 (SEQ ID NO:10).

**Table 3**

| Primer | SEQ ID NO | Sequence |
|---|---|---|
| 497c-cds9 | 12 | 5' GACGACGACAAGATCCCCCCCAGCTGCTGCATG 3' |
| 497c-cds-4 | 10 | 3'GAGGAGAAGCCCGGTctccagcgatgattcccatgtt 5' |

### 497C-T5

The truncated 497C-T4 was built by PCR amplification of the DNA using the vector pET30-497C-T2 as template, the 5' specific primer 497c-cdsll (SEQ ID NO:13), which permitted to eliminate the first and the second C-C bridges, the b, c and d1 domains, and to preserve the linear sequence corresponding to the d2 and e domains, and the 3' specific primer 497C-cds4 (SEQ ID NO:10).

**Table 4**

| Primer | SEQ ID NO | Sequence |
|---|---|---|
| 497c-cds11 | 13 | 5' GACGACGACAAGATGAACGAAACTGATTGTAATCCCC 3' |
| 497c-cds-4 | 10 | 3'GAGGAGAAGCCCGGTctccagcgatgattcccatgtt 5' |

For the N-terminal His-Tag and the C-terminal tail, the constructs were obtained using the following primers which totally eliminated the sequence of the protein 497C-T2.

**Table 5**

| Primer | SEQ ID NO | Sequence |
|---|---|---|
| Pet30 cds1 | 14 | 5'GACGACGACAAGATGGACCGGGCTTCTCCT 3' |
| pet30 cds2 | 15 | 5'GAGGAGAAGCCCGGTCTAGTTATTGCTCAGCGG 3' |

The amplified products corresponding to the truncated forms 497C-T3, 497C-T4, 497C-T5, and the C-terminal tail were cloned and produced as described for the protein 497C-T2.

### EXAMPLE 3: Test of inhibition of endothelial cell proliferation by 497-T2 in vitro

### Cell Culture

HUVEC cells were cultured to confluency in complete EGM2-MV medium (Cambrex) at 37°C and in 5% CO₂ humidified atmosphere. Cells were then collected by trypsine-EDTA digestion (Versene, Eurobio). After 5 min, the enzymatic reaction was stopped by adding 3 ml of the culture medium containing 5% FCS. Cells were then centrifuged at 220 g for 10 min at room temperature, washed twice with 5 ml of culture medium, suspended in complete culture medium, counted and adjusted to 50 000 cells/ml. One hundred µL per well were then distributed to a 96-well cell culture grade micro-plate (5 000 cells/well) and incubated with different concentrations of the purified protein 497C-T2 in Tris-HCl 20 mM buffer (pH 8), containing 150mM NaCl and urea 2M; this buffer was used as control.

After 42 hrs at 37°C, cell proliferation was measured using thiazolyl blue tetrazolium bromide (MTT) method. Briefly, MTT (Sigma) was dissolved in PBS at 5 mg/ml, the solution was filtered (0.22 µm) and 10 µl were added to each well of the 96-well micro-plates. After 3 hrs of incubation at 37°C, 5% CO₂ humidified atmosphere, the micro-plates were centrifuged at 220 x g for 10 min, the supernatant was discarded, and the crystals dissolved by the addition of 100 µl of DMSO to each well. The optical density (OD) at 570 nm was then measured using µQuant micro-plate reader (Bio-Tek Instrument gmbh, Colmar, France) coupled to the KC4 (Bio-Tek) software. The OD was corrected by subtracting blank-well OD values (the OD values obtained from wells without cells), and the inhibition of cell proliferation was measured relative to control (OD obtained from wells with untreated HUVEC representing the maximal proliferative response, i.e.100%).

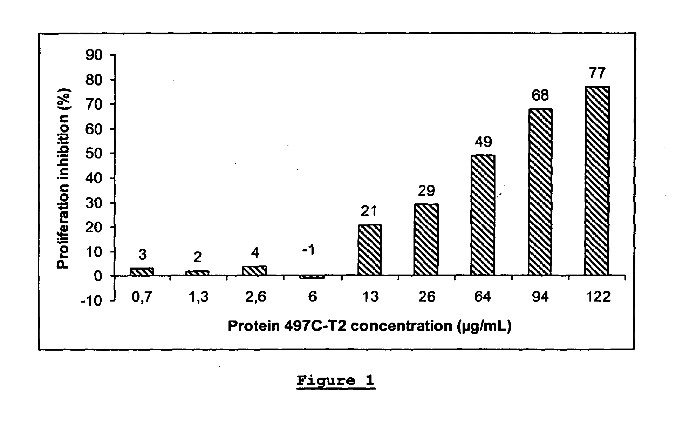

As shown in Figure 1, protein 497C-T2 inhibited human endothelial cell proliferation in a dose dependent manner. This inhibition represented 77% at 122µg (i.e. 5µM) of protein 497C-T2.

### EXAMPLE 4: Inhibition of in vitro angiogenesis by 497-T2, 497C-T3, 497C-T4 and 497C-T5

The purified proteins 497C-T2 497C-T3, 497C-T4 and 497C-T5 were tested *in vitro* on angiogenesis of HUVEC induced by FGF2 and VEGF on Matrigel.

24 wells plates were prepared with 250µL of BD Matrigel™/well and then incubated 30 minutes in incubator. HUVEC cells were then prepared as described in example 3 and 70 000 cells were seeded per well and incubated with different concentrations of the purified protein 497C-T2, 497C-T3, 497C-T4 or 497C-T5 in Tris-HCl 20 mM buffer (pH 8), containing 150mM NaCl and urea 2M; this buffer was used as control:
- Figure 2a: Control (Buffer Urea 2M)
- Figure 2b: 497C-T2 6.5µg/mL (0.27µM)
- Figure 2c: 497C-T2 13µg/mL (0.54µM)
- Figure 2d: 497C-T2 26µg/mL (1.08µM)
- Figure 2e: 497C-T2 48µg/mL (2µM)
- Figure 2f: 497C-T3 51µg/mL (2.55µM)
- Figure 2g: 497C-T4 55µg/mL (3.18µM)
- Figure 2h : 497C-T5 56µg/mL (3.35µM)
- Figure 2i : C-terminal tail 53µg/mL (4.14µM)

As shown in Figures 2b, 2c, 2d and 2e, protein 497C-T2 inhibited *in vitro* angiogenesis in a dose-dependent manner.

Moreover, as shown in figures 2f, 2g and 2h, the truncated forms 497C-T3, 497C-T4 and 497C-T5 showed different levels of activity: 497C-T3 (Fig. 2f) and 497C-T4 (Fig. 2g) were almost as efficient as 497C-T2 for the anti-angiogenic activity, suggesting that the a, b and c LEL domains are not necessary for the anti-angiogenic activity of protein 497C-T2. To the contrary, 497C-T5 (fig 2h) showed only a residual inhibitory activity, while the C-terminal tail was totally inactive (Fig. 2i).

### EXAMPLE 5: Inhibition of the migration of human endothelial cells by 497-T2, 497C-T3, 497C-T4 and 497C-T5

Cell migration was tested by the wound assay described by Sato and Rifkin (J Cell Biol. 1988; 107: 1199) with few modifications. HUVEC grown in growth medium EGM-2MV (Cambrex) were seeded in 24-well plates at 80 000 cells per well in 500µL of growth medium and grown to confluence at 37°C in a humidified atmosphere containing 5% CO₂. Cells were scrapped with a plastic tip on one line only. After wounding, the culture medium was changed for fresh medium (control, figure 3a) or fresh medium supplemented with:
- Figure 3b: 497C-T2 20µg/mL (0.83µM)
- Figure 3c: 497C-T2 40µg/mL (1.66µM)
- Figure 3d: 497C-T3 48µg/mL (2.4µM)
- Figure 3e: 497C-T4 60µg/mL (3.52µM)
- Figure 3f: 497C-T5 61µg/mL (3.65µM)
- Figure 3g : C-terminal tail 63µg/mL (4.92µM) After 18 hours of culture, cells were observed and photographed under the inverted microscope (Analysis, Olympus, Rungis, France).

As shown in Figures 3b and 3c, protein 497C-T2 inhibited human endothelial cells migration in a dose dependent manner.

Moreover, as shown in Figures 3d, 3d, 3e and 3f the truncated forms 497C-T3, 497C-T4 and 497C-T5 showed different levels of activity. The truncated form 497C-T3 inhibited cell migration similarly to the protein 497C-T2 (Fig. 3d). To the contrary, the truncated form 497C-T4 (Fig. 3e) was less active than 497C-T2, 497C-T5 (Fig. 3f)

showed very limited anti-migratory activity, and the C-terminal tail (Fig. 3g) was totally inactive. These results thus suggested that the a and b LEL domains are not necessary for the anti-migratory activity of protein 497C-T2.

### EXAMPLE 6: Expression of protein 497C in tumor samples

A number of different human tumor samples were screened for the expression of the gene 497C. For each pathological sample, the periphery of the tumor was separated from the core of the tumor, and the expression of the gene 497C in these two area was compared after mRNA extraction followed by RT-PCR.

### Kidney tumor samples

19 pathological biopsies from kidney tumors were analysed. In 13 out of 19 patients, the expression of 497C was much higher in the core than in the periphery of the tumor.

### Lung tumor samples

40 pathological biopsies from human lung tumors were analysed. In 37 out of 40 patients, the expression of 497C was much higher in the periphery than in the core of the tumor. There was also a close relationship between the level of expression of 497C at the periphery of the tumor and the patient's nod status (i.e. the metastasis potential). The anatomical examination of the samples also revealed that the periphery of the tumor was much more vascularised than the core of the tumor (as established for lung cancer in general).

### Colon tumor samples

33 pathological biopsies from human colon tumors were analysed. In 25 out of 33 patients, the expression of 497C was much higher in the periphery than in the core of the tumor.

### EXAMPLE 7: Test of 497C-T2 on NCI H460 human tumor in swiss nude mice in vivo

### Preparation of NCI H460 cells

NCI H460 cells were cultured as adherent cells in complete RPMI 1640 medium (Ref. CM1RPM08-01, batch No. 623615, Eurobio, France) containing 2 mM L-Glutamine, adjusted to 4.5 g/L glucose (Ref. G7528, batch No. 033K0121 , Sigma, France) and supplemented with 10 mM HEPES (Ref. H0887, batch No. 113K2338, Sigma, France), 1.0 mM sodium pyruvate (Ref. CSTVAT00-0U, batch No. 520818, Sigma, France) and 10% fetal calf serum (FCS; Ref. CVFSVF00-01, batch No. S13021, Eurobio, France) under a 37°C, 5% CO₂ humidified atmosphere. They were amplified in 75 cm²-flasks to reach 90x10⁶ cells.

At D0, NCI H460 cells (human lung carcinoma) were collected from 75 cm²-flasks by removing the medium and adding 3 ml of trypsine-EDTA (Ref. CEZTDA00-0U, batch No. 633920, Eurobio, France). After 5 min of incubation at 37°C, cells were detached from the plastic and the enzymatic reaction was stopped by adding 3 ml of RPMI 1640 medium containing 10% fetal calf serum. Cells were then centrifuged at 700 g for 5 min at room temperature. They were resuspended in serum-free RPMI 1640 culture medium containing L-Glutamine (2 mM), glucose (4.5 g/1), sodium pyruvate (1 mM) and buffered with HEPES (10 mM). Cells were counted and the number of viable NCI H460 cells was > 99%. The number of cells was then adjusted to 25.10⁶ cells/ml in serum-free medium.

### Tumor induction

Thirty healthy female Swiss Nude mice were anesthetized by IP injection of Ketamine-Xylazine (80 mg/kg-12 mg/kg; Ref. K-113, Sigma, France). NCI H460 cells (5.10⁶ cells/mouse in 200 µL of serum-free medium) were then implanted subcutaneously in the right flank of each mouse.

### Treatment schedule

At D12 post-implantation of the NCI H460 cells, the thirty mice were randomized into six groups of 5 mice. Tumor volumes had reached 228 to 468 mm³ and mean tumor volumes were not statistically different between groups after randomization.

The treatment schedule, starting D12 and ending D28, is summarized in Table 6:
- Animals of group 1 were treated with the vehicle solution (Batch C): Tris-HCl pH 7.5, 2M Urea, 150mM NaCl, 0.1mM CaCl₂,
- Animals of group 2 were treated with a solution of cisplatin in physiological serum 0.5 ml/mL (GDDP, cis-diamineplatinum(II) dichloride, Ref. P4394, batch No. 014K0993, Sigma, France, purity 100%, MW. 300),
- Animals of groups 3, and 4 were treated with the vehicle solution supplemented with 1mg/mL of the test substance 497C-T2 (Batch A),
- Animals of group 5 were treated with the vehicle solution supplemented with 1mg/mL of the test substance 497C-T2 (Batch A), and further received CDDP.
- Animals of group 6 were treated with the vehicle solution supplemented with 1.8mg/mL of the test substance 497C-T2 (Batch B), and further received CDDP.

All solutions were injected IP. Injections in groups 1, 2, 3, 5 and 6 were performed according to the schedules Q2DX8, i.e. 1 quantity every two days, eight times. Injections in group 4 were performed according to the schedules Q1DX16, i.e. 1 quantity every day, sixteen times.

CDDP was resuspended in sterile physiological serum at a concentration of 0.5mg/mL and injected IP at a concentration of 5mg/kg at a volume of 10mL/kg, according to the treatment schedule Q2DX8.

Mice were observed for 2h post-injection. Ketamine/Xylazine (80 mg/kg - 12 mg/kg; Ref. K-113, Sigma, France) was used to anaesthetize the animals before sacrifice by cervical dislocation.

### Monitoring of the mice

Animals have been observed daily. Modification in animal behavior has been reported in the laboratory notebook. Body weights and tumor volumes were recorded every two days until the end of the experiment.

Data outlined below were calculated:
- Tumor growth curves were drawn using the mean tumor volumes (MTV),
- Mean Relative tumor volume (MRTV) was calculated as the ratio between the MTV at time t and the volume at the time of injection (t=D12),
- Tumor growth inhibition (T/C, %) was evaluated as the ratio of the median tumor volumes of treated groups *versus* vehicle group.

**Table 6**

| **Group** | **Animals n** | **Treatment** | **Administration route** | **Treatment dose (mg/kg/adm)** | **Administration volume** | **Treatment schedule** |
|---|---|---|---|---|---|---|
| **1** | 5 | Vehicle (Batch C) | IP | 0 | 10 ml/kg | Q2DX8 |
| **2** | 5 | Cisplatin | IP | 5 | | Q2DX8 |
| **3** | 5 | 497C-T2 (Batch A) | IP | 10 | | Q2DX8 |
| **4** | 5 | 497C-T2 (Batch A) | IP | 10 | | Q1DX16 |
| **5** | 5 | 497C-T2 (Batch A) & Cisplatin | IP | 10 5 | | Q2DX8 |
| | | | | | | Q2DX8 |
| **6** | 5 | 497C-T2 (Batch B) & | IP | 18 | | Q2DX8 |
| | | Cisplatin | | 5 | | Q2DX8 |

**Table 7: Mean body weight (MBW) of mice bearing NCI H-460 tumors treated with the vehicle, CDDP at 5 mg/kg (schedule Q2DX8, G2), 497C-T2 at 10.0 mg/kg (schedule Q2DX8, G3), 497C-T2 at 10.0 mg/kg (schedule Q1DX16, G4), combined 497C-T2 at 10.0 mg/kg and CDDP at 5 mg/kg (schedule Q2DX8, G5), and combined 497C-T2 at 18.0 mg/kg and CDDP at 5 mg/kg (schedule Q2DX8, G6) at D12 and D28.**

| **Group** | **Test substance** | **Treatment dose (mg/kg**) | **MBW at D12(g)** | **MBW at D28 (g)** | **MBWC D12-D28 (g)** |
|---|---|---|---|---|---|
| **1** | Vehicle-Batch C | 0 | 22.53 ± 0.79 | 23.72 ± 0.72 | 1.18 ± 0.79 |
| **2** | Cisplatin | 5.00 | 21.29 ± 0.41 | 18.74 ± 1.59 | -2.55 ± 1.83 |
| **3** | 497C-T2-Batch A | 10.0 | 24.06 ± 1.59 | 25.70 ± 1.12 | 1.63 ± 1.59 |
| **4** | 497C-T2-Batch A | 10.0 | 21.76 ± 1.63 | 23.68 ± 2.17 | 1.91 ± 1.36 |
| **5** | 497C-T2-Batch A + Cisplatin | 10.0 5.00 | 23.28 ± 2.03 | 20.19 ± 0.66 | -3.09 ± 2.68 |
| **6** | 497C-T2-Batch B + Cisplatin | 18.0 5.00 | 23.08 ± 1.78 | 19.00 ± 2.00 | -4.08 ± 0.71 |

### Statistical studies

Statistical analyses of tumor volumes (V), time to reach `V', tumor-doubling time (DT), relative tumor volume (RTV) and tumor growth inhibition (T/C) were performed for all groups. Data are expressed as mean±SD. Groups of data were normally distributed. Univariate analysis were performed to assess differences between groups. Statistical significance was then determined using the Student's t test. A P<0.05 was considered as statistically significant. The Statistical analysis was performed using XLSTAT (Addinsoft, France).

### Body weight monitoring

As shown in table 7, the vehicle had no impact on the body weight: mouse behavior and body weight gain were normal and no animal died prematurely. No toxicity was observed during the course of the treatment with the test substance 497C-T2 at both doses tested (10 and 18 mg/kg). In contrast, an important toxicity was observed in groups 2, 5 and 6 treated with CDDP (from -12 to -18% body weight loss; p<0.05).

The results of mean tumor volume *(MTV)*, mean relative tumor volume *(MRTV)*, tumor volume and tumor growth parameters are shown in Figures 4, 5 and in Table 8 and Table 9.

As shown in table 8, the MTV was decreased at D28 in mice of group 2 treated with CDDP (1440 ± 1097 mm³) compared to mice of the vehicle group 1 (4441 ± 1135 mm³). The MTV at D28 was also decreased in groups 3 and 4 treated with the test substance 497C-T2 at 10 mg/kg with 1 injection per day (2482 ± 2075 mm³) and 1 injection every other day (3167 ± 1681 mm³), respectively. A massive reduction of the MTV was observed in animals from group 5 (807 ± 692 mm³) and group 6 (639 ± 416 mm³) compared to group 1, the vehicle treated animals (4441 ± 1135 mm³).

The T/C ratio, which is a parameter of tumor growth inhibition, represented 32% in mice from group 2 at D28 demonstrating that CDDP reduces by 68% tumor size compared to the vehicle-treated group 1. T/C was 56% in mice from for group 3 and 71% in mice from group 4, revealing a moderate anti-tumoral activity of the test substance when used as a monotherapy. When combined with CDDP however, T/C fell to 18% and 14% in mice from groups 5 and 6, respectively. This demonstrates that 497C-T2 has a potent anti-tumoral activity when combined a cytotoxic agent such as CDDP.

The tumor size doubling time (DT) was 1.23 ± 0.49 days in the vehicle-treated group 1. The DT increased 4-fold in mice of group 2 treated with CDDP alone (5.12 ± 2.89 days). For groups 3 and 4, the DT (4.08 ± 1.4 and 5.9 ± 1.21 days, respectively) was similar to that of group 2. This indicates that 497C-T2 is as potent as CDDP to reduce tumor activity when used as monotherapy. But most importantly, bi-therapy using 497C-T2 and CDDP increased 20-fold DT in animals from groups 5 and 6 (25.02 ± 29.89 and 20.07 ± 9.28 days, respectively) compared to the DT measured in mice from the vehicle-treated group 1. This further confirms the potent anti-tumoral activity of the tested substance 497C-T2 when used alone or in combination with CDDP.

As shown in table 9, MRTV measures confirmed that animal of group 6 presented the strongest decrease of the tumor volume, at both concentration tested (group 5,6). Treatment with 497C-T2 alone led to a MRTV at D28 of 7,37

(group 3) or 6,76 (group 4) and treatment with cisplatin led to a MRTV of 4,88 (group 2).

All these results confirm that 497C-T2 is a potent anti-tumor agent, either used alone or in a synergistic combination with cisplatin.

### EXAMPLE 8: Test of 497C-T2 on non-small human cell lung cancer (CALU-6) xenograft model in swiss nude mice in vivo

### Prepa ration of CALU-6 cells

CALU-6 cells were cultured as adherent cells in complete EMEM medium (Ref. CM1MEM18-01, batch No. 462502, Eurobio, France) 10% fetal calf serum (FCS; Ref. CVFSVFOO-01, batch No. S13021, Eurobio, France) under a 37°C, 5% CO₂ humidified atmosphere. They were amplified in 75 cm²-flasks to reach 90x10⁶ cells.

At D0, CALU-6 cells (human lung carcinoma) were collected from 75 cm²-flasks by removing the medium and adding 3 ml of trypsine-EDTA (Ref. CEZTDA00-0U, batch No. 633920, Eurobio, France). After 5 min of incubation at 37°C, cells had detached from the plastic and the enzymatic reaction was stopped by adding 3 ml of EMEM medium containing 10% fetal calf serum. Cells were then centrifuged at 700 g for 5 min at room temperature. They were resuspended in serum-free EMEM culture medium. Cells were counted and viability assessment by Trypan Blue exclusion (Ref. CSTCOL03-OU, batch No. 434511, Eurobio, France). The number of viable CALU-6 cells was > 99%. The number of cells was then adjusted to 25x10⁶ cells/ml in serum-free medium.

**Table 8: Monitoring of human NCI H460 tumor growth in Nude mice after IP treatment with vehicle, 497C-T2 (10 mg/kg and 18 mg/kg) and CDDP (5 mg/kg). MTV: mean tumor volume; T/C: treated/vehicle tumor volume ratio; DT: doubling time of tumor volume. a : p<0.05 vs G1 - b : p<0.05 vs G2 -c : p<0.05 vs G3 - d : p<0.05 vs G4.**

| **Group** | **Test substance** | **Treatment dose (mg/kg)** | **MTV at D14 (mm³)** | **MIV at D24 (mm³)** | **MTV at D28 (mm³)** | **T/C at T/C at D14 (%)** | **D24 (%)** | **T/Cat D28 (%)** | **DT (days)** |
|---|---|---|---|---|---|---|---|---|---|
| **1** | Vehicle-Batch C | 0 | 999.64 ± 443.46 | 3222.36 ± 494.51 | 4440.68 ± 1135.16 | 100 | 100 | 100 | 1.23 ± 0.49 |
| **2** | CDDP | 5.0 | 513.35 ± 333.36 | 1083.74 ± 899.44 | 1440.18 ± 1097.29 ^{a} | 51.53 | 33.63 | 32.43 | 5.12 ± 2.89 |
| **3** | 49-7C-T2-Batch A- | 10.0 | 571.00 ± 306.73 | 1955.02 ± 1687.55 | 2481.61 ± 2075.18 | 57.12 | 60.67 | 55.88 | 4.08 ± 1.4 |
| **4** | 497C-T2-Batch A- | 10.0 | 468.85 ± 250.00 | 2289.22 ± 1170.70 | 3167.34 ± 1680.99 | 46.90 | 71.04 | 71.32 | 5.9 ± 1.21 |
| **5** | 497C-T2-Batch A-+CDDP | 10.0 | 388.79 ± 315.14 | 671.93 ± 570.47 ^{a} | 807.43 + 807.43 ± 692.22 ^{a,c} | 38.89 | 20.85 | 18.18 | 25.02 ± 29.89 |
| | | 5.0 | | | | | | | |
| **6** | 497C-T2-Batch B-+CDDP | 18.0 | 459.45 ± 166.55 ^{a} | 561.71 ± 218.34 ^{a,b,c,d d} | 638.74 ± 415.53 ^{a, b, c, d} | 45.96 | 17.43 | 14.38 | 20.07 ± 9.28 |
| | | 5.0 | | | | | | | |

**Table 9: Mean relative tumor volume (MRTV) of animals bearing the NCI H460 tumor and treated with vehicle (Group 1), CDDP alone (Group 2), 497C-T2 administered every other day (Group 3) or once daily (Group 4), and CDDP combined with 497C-T2 at the lower (Group 5) and higher dose (Group 6).**

| | D12 | D14 | D16 | D18 | D20 | D22 | D24 | D26 | D28 |
|---|---|---|---|---|---|---|---|---|---|
| **Group 1** | 1.00 | 3.18 | 4.39 | 6.87 | 9.28 | 11.61 | 9.73 | 14.15 | 19.50 |
| **Group 2** | 1.00 | 1.59 | 1.74 | 2.30 | 2.29 | 3.39 | 3.67 | 3.67 | 4.88 |
| **Group 3** | 1.00 | 1.37 | 1.70 | 2.46 | 2.81 | 3.30 | 3.22 | 5.80 | 7.37 |
| **Group 4** | 1.00 | 1.08 | 1.00 | 1.69 | 2.63 | 3.08 | 3.08 | 4.89 | 6.76 |
| **Group 5** | 1.00 | 1.01 | 1.18 | 1.34 | 1.75 | 2.18 | 1.00 | 2.04 | 2.45 |
| **Group 6** | 1.00 | 1.24 | 1.06 | 0.89 | 1.07 | 1.18 | 1.17 | 1.30 | 1.48 |

### Tumor induction

Thirty healthy female Swiss *Nude* mice were anesthetized by IP injection of Ketamine-Xylazine (80 mg/kg-12 mg/kg; Ref. K-113, Sigma, France). CALU-6 cells 5x10⁶ cells/mouse in 200 µl of serum-free medium) were then implanted subcutaneously in the right flank of each mouse. Mice were observed for 2h post-implantation.

### Treatment schedule

At D12 post-implantation of the CALU-6 cells, the thirty mice were randomized into six groups of 5 mice. Tumor volumes had reached 228 to 468 mm³ and mean tumor volumes were not statistically different between groups after randomization.

The treatment schedule, starting D12 and ending D28, is summarized in Table 10.
- Animals of group 1 were treated with the vehicle solution (Tris-HCl pH 7.5, 2M Urea, 150mM NaCl, 0.1mM CaCl₂) (Batch C);
- Animals of group 2 were treated with a solution of cisplatin in physiological serum 0.5 mh/mL(CDDP, cis-diamineplatinum(II) dichloride, Ref. P4394, batch No. 014K0993, Sigma, France, purity 100%, MW. 300),
- Animals of group 3 were treated with the vehicle supplemented with the test substance 497C-T2 at a dose of 10 mg/kg.
- Animals of group 4 were treated with the vehicle supplemented with the test substance 497C-T2 at a dose of 10 mg/kg, and further received CDDP.

Injections in groups 1, 2, 3 and 4 were performed according to the schedules Q2DX8, i.e. 1 quantity every two days, eight times.

CDDP was resuspended in sterile physiological serum at a concentration of 0.5mg/mL and injected IP at a concentration of 5mg/kg at a volume of 10mL/kg, according to the treatment schedule Q2DX8.

Mice were observed for 2 hours post-injection. Ketamine/Xylazine (80 mg/kg - 12 mg/kg; Ref. K-113, Sigma, France) was used to anaesthetize the animals before sacrifice by cervical dislocation. For all animals, the tumor size was measured twice a week with calipers. The tumor volume (mm³) was measured according to the formula: (length x width²)/2 (4).

### Statistical studies

Mean tumor volumes (MTV), mean relative tumor volume (MRTV) and tumor growth inhibition (T/C) were calculated as for example 7.

### Body weight

As shown in table 11, the vehicle had no impact: mouse behavior and body weight gain were normal and no animal died prematurely (excepted mouse 5 of group 1). No toxicity was observed during the course of the treatment with the test substance 497C-T2 at the dose of 10 mg/kg, a slight body weight gain was observed (+1,44g).

In contrast, an important toxicity was observed in groups 2, 4 treated with CDDP. (12.6% and 8,7% body weight loss respectively). The difference between group 1 versus 2 and 4 and group 3 versus 2 and 4 was statistically significant (p<0,0001) but the difference between group 2 and 4 was not statistically significant.

**Table 10**

| **Group** | **Animals n** | **Treatment** | **Administration route** | **Treatment dose (mg/kg/adm)** | **Administration volume** | **Treatment schedule** |
|---|---|---|---|---|---|---|
| **1** | 5 | Vehicle | IP | 0 | 10 ml/kg | Q2DX8 |
| **2** | 5 | Cisplatin | IP | 5 | | Q2DX8 |
| **3** | 5 | 497C-T2 | IP | 10 | | Q2DX8 |
| **4** | 5 | 497C-T2 & Ciplatine | IP | 10 | | Q2DXB |
| | | | | 5 | | Q2DX8 |

**Table 11: Mean body weight (MBW) of mice bearing CALU-6 tumors treated with the vehicle, CDDP at 5 mg/kg (schedule Q2DX8, G2), 497C-T2 at 10.0 mg/kg (schedule Q2DX8, G3), combined 497C-T2 at 10.0 mg/kg and CDDP at 5 mg/kg (schedule Q2DX8, G4) at D12 and D28.**

| **Group** | **Test substance** | **Treatment dose (mg/kg)** | **MBW at D12 (g)** | **MBW at D28 (g)** | **MBWC D12-D28 (g)** |
|---|---|---|---|---|---|
| **1** | Vehicle | 0 | 22.35 ± 1.17 | 24.40 ± 1.14 | + 2,04 |
| **2** | Cisplatin | 5.00 | 21. 39 ± 0.85 | 18.69 ± 1.97 | -2.70 |
| **3** | 497C-T2 | 10.0 | 21.67 ± 1.32 | 23.11 ± 1.97 | +1.44 |
| **4** | 497C-T2+ Cisplatin | 10.0&5.00 | 21.94 ± 0.74 | 19.84 ± 1.89 | -2,11 |

The results of mean tumor volume *(MTV)*, mean relative tumor volume *(MRTV)*, tumor volume and tumor growth parameters are shown in Figures 6, 7 and in Table 12 and Table 13.

The MTV was decreased at D28 in mice of group 2 treated with CDDP (742.44 ± 215.85 mm³) compared to mice of the vehicle group 1 (1 233,44 ± 663,82 mm³). The MTV at D28 was also decreased in group 3 treated with the test substance 497C-T2 at 10 mg/kg with 1 injection per two days (813,70 ± 439,00 mm³). These results were confirmed by the analysis of the MRTV at D28 (table 13). A massive reduction of the MTV was observed in animals from group 4 (430.89 ± 290.89 mm³) compared to group 1, the vehicle treated animals (1 233,44 ± 663,82 mm³).

The difference between group 1 and the 2 groups treated with the test substance reach the statistical significativity (p< 0,0001 vs 4 - p = 0,003 vs 3). The difference between group 2 and 4 was also significant (p=0,001). In contrast no statistical difference was observed between group 2 (CDDP alone) and group 3 (497c-T2 alone). The first statistical significativity between group 1 and treated group was observed respectively at D22 for groups 3 and D20 for group 4. These results were confirmed by the analysis of the MRTV at D28 (table 13). An important reduction of the MTV was observed in animals from group 4 (430,89 ± 290,89 mm³) compared to group 1, the vehicle treated animals (1 233,44 ± 663,82 mm³).

**Table 12: Growth inhibition based on T/C ratio**

| **T/C ratio (%)** | | | | | | | | |
|---|---|---|---|---|---|---|---|---|
| **Day** | **D14** | **D16** | **D18** | **D20** | **D22** | **D24** | **D26** | **D28** |
| **G2** | -70% | 24% | -6% | 17% | 11% | 15% | 45% | 27% |
| **G3** | 10% | 33% | 7% | 13% | 26% | 24% | 39% | 27% |
| **G4** | 22% | 39% | 8% | 28% | 30% | 41% | 60% | 64% |

The T/C ratio (table 12), which is a parameter of tumor growth inhibition, reveals a slight anti-tumoral activity of the test substance when used as a monotherapy as it reduces by 27% tumor size compared to the vehicle-treated group 1. However, when combined with CDDP, the inhibition rate reach 64% reduction of tumor size relative to the vehicle-treated group 1. These results directly demonstrate that 497C-T2 has a potent anti-tumoral activity when it was used in combination with a cytotoxic agent such as CDDP.

**Table 13: Mean Relative tumor volume (MRTV) of animals bearing NCI H-460 cells and treated with vehicle (group 1), CDDP alone (Group 2), 497C-T2 (10.0 mg/kg) (Group 3), or combined with 497C-T2 and CDDP (Group 4) according to the scheduled treatment Q2DX8.**

| **MRTV** | | | | | | | | | |
|---|---|---|---|---|---|---|---|---|---|
| **Group** | **D12** | **D14** | **D16** | **D18** | **D20** | **D22** | **D24** | **D26** | **D28** |
| **1** | 1 | 1,83 | 3,17 | 2,51 | 3,68 | 4,16 | 5,68 | 7,49 | 8,24 |
| **2** | 1 | 3,11 | 2,39 | 2,66 | 3,08 | 3,70 | 4,84 | 4,11 | 5,98 |
| **3** | 1 | 1,64 | 2,11 | 2,34 | 3,22 | 3,09 | 4,31 | 4,60 | 6,02 |
| **4** | 1 | 1,42 | 1,92 | 2,32 | 2,66 | 2,89 | 3,33 | 2,96 | 2,96 |

As shown in table 13, MRTV reached 2,96 at D28 for the animals of group 4, which confirm the synergistic efficacy of 497C-T2 with Cisplatin. Moreover, cisplatin used alone (group 2) or 497C-T2 used alone (group 3) showed very close MRTV at D28, suggesting that 497C-T2 is also a potent mono-therapy anti-tumor agent.

### SEQUENCE LISTING

<110> GENE SIGNAL INTERNATIONAL SA
<120> Anti-tumor drug, medicament, composition and use thereof
<130> BET070030
<160> 15
<170> PatentIn version 3.3
<210> 1
   <211> 1801
   <212> DNA
   <213> Homo sapiens
<400> 1
   cttccccctc ccgcgcgccc gcccgccgcc tgccgccgcc gccgccgccg ccggagctct 60
   gtagtatggc atcgaggaga atggagacca aacctgtgat aacctgtctc aaaaccctcc 120
   tcatcatcta ctccttcgtc ttctggatca ctggggtgat cctgctggct gttggagtct 180
   ggggcaaact tactctgggc acctatatct cccttattgc cgagaactcc acaaatgctc 240
   cctatgtgct catcggaact ggcaccacta ttgttgtctt tggcctgttt ggatgctttg 300
   ctacatgtcg tggtagccca tggatgctga aactgtatgc catgtttctg tccctggtgt 360
   tcctggctga gctcgtagct ggcatttcag ggtttgtgtt tcgtcatgag atcaaggaca 420
   ccttcctgag gacttacacg gacgctatgc agacttacaa tggcaatgat gagaggagcc 480
   gggcagtgga ccatgtgcag cgcagcctga gctgctgtgg tgtgcagaac tacaccaact 540
   ggagcaccag cccctacttc ctggagcatg gcatcccccc cagctgctgc atgaacgaaa 600
   ctgattgtaa tccccaggat ctacacaatc tgactgtggc cgccaccaaa gttaaccaga 660
   agggttgtta tgatctggta actagtttca tggagactaa catgggaatc atcgctggag 720
   tggcgtttgg aatcgcattc tcccagttaa ttggcatgct gctggcctgc tgtctgtccc 780
   ggttcatcac ggccaatcag tatgagatgg tgtaaggaga agtctttcaa gaatgacgga 840
   ataagagacc tgttttaaaa aggaactgca gcaatctttg aaagacttcc aaagaatgtt 900
   agagcacagt acataataca cttgccctgc tccctctccc ccttacccca caacgtgcaa 960
   ctgacactcc cacccagtct ctgctccacc tttcagccca cgtcacgtgt agtgtccatt 1020
   ttgtgaagcc ctgttgtgcc acagagtgta gccaggtccc cctgcagcta gtcctagtga 1080
   acctcacccc gaggccctgc atgggccagc ccctccatct gtacttggtc caactgcaac 1140
   tcatcatcgg tgactggtta tcacaccatc gctggcccct ttgggccctg catgtagtgt 1200
   gggaggctcc tgttagctcc tcactgtggt aaatgccaca cacctttaag tagataagca 1260
   gacgatagtt atctgttctt ttgacttaat ctcatttggt ttgattttcc ctctactaag 1320
   gctttcctac cttcttcagg ctgcctaaga catgtaacga aacacttcaa taattgtcca 1380
   tgaggagaaa aaaagcatgt gtcatgcatg aaggaaactg aacttgaggt ggcctccttg 1440
   cttgttacat acctgggtat gtgtaggcag tttagtgcat ctttgcctct cggttgaaac 1500
   ctgtataacc ctgttacaaa gctgtgttgt tgcttcttgt gaaggccatg atattttgtt 1560
   tttccccaat taattgctat tgtgttattt tactacttct ctctgtattt tttcttgcat 1620
   tgacattata gacattgagg acctcatcca aacaatttaa aaatgagtgt gaagggggaa 1680
   caagtcaaaa tatttttaaa agatcttcaa aagtaatgcc tctgtctagc atgccaacaa 1740
   gaatgcattg atattgtgaa catttgtgat atatgtatta ataaatagag caattacaag 1800
   c 1801
<210> 2
   <211> 244
   <212> PRT
   <213> Homo sapiens
<400> 2
   Met Glu Thr Lys Pro Val Ile Thr Cys Leu Lys Thr Leu Leu Ile Ile
   1               5                   10                  15
   Tyr Ser Phe Val Phe Trp Ile Thr Gly Val Ile Leu Leu Ala Val Gly
               20                  25                  30
   Val Trp Gly Lys Leu Thr Leu Gly Thr Tyr Ile Ser Leu Ile Ala Glu
           35                  40                  45
   Asn Ser Thr Asn Ala Pro Tyr Val Leu Ile Gly Thr Gly Thr Thr Ile
       50                  55                  60
   Val Val Phe Gly Leu Phe Gly Cys Phe Ala Thr Cys Arg Gly Ser Pro
   65                  70                  75                  80
   Trp Met Leu Lys Leu Tyr Ala Met Phe Leu Ser Leu Val Phe Leu Ala
                   85                  90                  95
   Glu Leu Val Ala Gly Ile Ser Gly Phe Val Phe Arg His Glu Ile Lys
               100                 105                 110
   Asp Thr Phe Leu Arg Thr Tyr Thr Asp Ala Met Gln Thr Tyr Asn Gly
           115                 120                 125
   Asn Asp Glu Arg Ser Arg Ala Val Asp His Val Gln Arg Ser Leu Ser
       130                 135                 140
   Cys Cys Gly Val Gln Asn Tyr Thr Asn Trp Ser Thr Ser Pro Tyr Phe
   145                 150                 155                 160
   Leu Glu His Gly Ile Pro Pro Ser Cys Cys Met Asn Glu Thr Asp Cys
                   165                 170                 175
   Asn Pro Gln Asp Leu His Asn Leu Thr Val Ala Ala Thr Lys Val Asn
               180                 185                 190
   Gln Lys Gly Cys Tyr Asp Leu Val Thr Ser Phe Met Glu Thr Asn Met
           195                 200                 205
   Gly Ile Ile Ala Gly Val Ala Phe Gly Ile Ala Phe Ser Gln Leu Ile
       210                 215                 220
   Gly Met Leu Leu Ala Cys Cys Leu Ser Arg Phe Ile Thr Ala Asn Gln
   225                 230                 235                 240
   Tyr Glu Met Val
<210> 3
   <211> 112
   <212> PRT
   <213> Homo sapiens
<400> 3
   Ile Ser Gly Phe Val Phe Arg His Glu Ile Lys Asp Thr Phe Leu Arg
   1               5                   10                  15
   Thr Tyr Thr Asp Ala Met Gln Thr Tyr Asn Gly Asn Asp Glu Arg Ser
               20                  25                  30
   Arg Ala Val Asp His Val Gln Arg Ser Leu Ser Cys Cys Gly Val Gln
           35                  40                  45
   Asn Tyr Thr Asn Trp Ser Thr Ser Pro Tyr Phe Leu Glu His Gly Ile
       50                  55                  60
   Pro Pro Ser Cys Cys Met Asn Glu Thr Asp Cys Asn Pro Gln Asp Leu
   65                  70                  75                  80
   His Asn Leu Thr Val Ala Ala Thr Lys Val Asn Gln Lys Gly Cys Tyr
                   85                  90                  95
   Asp Leu Val Thr Ser Phe Met Glu Thr Asn Met Gly Ile Ile Ala Gly
               100                 105                 110
<210> 4
   <211> 74
   <212> PRT
   <213> Homo sapiens
<400> 4
   Gln Arg Ser Leu Ser Cys Cys Gly Val Gln Asn Tyr Thr Asn Trp Ser
   1               5                   10                  15
   Thr Ser Pro Tyr Phe Leu Glu His Gly Ile Pro Pro Ser Cys Cys Met
               20                  25                  30
   Asn Glu Thr Asp Cys Asn Pro Gln Asp Leu His Asn Leu Thr Val Ala
           35                  40                  45
   Ala Thr Lys Val Asn Gln Lys Gly Cys Tyr Asp Leu Val Thr Ser Phe
       50                  55                  60
   Met Glu Thr Asn Met Gly Ile Ile Ala Gly
   65                  70
<210> 5
   <211> 49
   <212> PRT
   <213> Homo sapiens
<400> 5
   Ile Pro Pro Ser Cys Cys Met Asn Glu Thr Asp Cys Asn Pro Gln Asp
   1 5 10 15
   Leu His Asn Leu Thr Val Ala Ala Thr Lys Val Asn Gln Lys Gly Cys
   20 25 30
   Tyr Asp Leu Val Thr Ser Phe Met Glu Thr Asn Met Gly Ile Ile Ala
   35 40 45
   Gly
<210> 6
   <211> 43
   <212> PRT
   <213> Homo sapiens
<400> 6
   Met Asn Glu Thr Asp Cys Asn Pro Gln Asp Leu His Asn Leu Thr Val
   1 5 10 15
   Ala Ala Thr Lys Val Asn Gln Lys Gly Cys Tyr Asp Leu Val Thr Ser
   20 25 30
   Phe Met Glu Thr Asn Met Gly Ile Ile Ala Gly
   35 40
<210> 7
   <211> 1051
   <212> DNA
   <213> Homo sapiens
<220>
   <221> misc_feature <222> (59)..(59)
   <223> n is a, c, g, or t
<220>
   <221> misc_feature
   <222> (702)..(702)
   <223> n is a, c, g, or t
<220>
   <221> misc_feature
   <222> (832)..(832)
   <223> n is a, c, g, or t <220>
   <221> misc_feature
   <222> (841)..(841)
   <223> n is a, c, g, or t
<220>
   <221> misc_feature
   <222> (858)..(858)
   <223> n is a, c, g, or t
<220>
   <221> misc_feature
   <222> (862)..(862)
   <223> n is a, c, g, or t
<220>
   <221> misc_feature
   <222> (869)..(869)
   <223> n is a, c, g, or t
<220>
   <221> misc_feature
   <222> (891)..(891)
   <223> n is a, c, g, or t
<220>
   <221> misc_feature
   <222> (910)..(910)
   <223> n is a, c, g, or t
<220>
   <221> misc_feature
   <222> (918)..(918)
   <223> n is a, c, g, or t
<220>
   <221> misc_feature
   <222> (922)..(922)
   <223> n is a, c, g, or t
<220>
   <221> misc_feature
   <222> (942)..(942)
   <223> n is a, c, g, or t
<220>
   <221> misc_feature
   <222> (965)..(965)
   <223> n is a, c, g, or t
<220>
   <221> misc_feature
   <222> (991)..(991)
   <223> n is a, c, g, or t
<220>
   <221> misc_feature
   <222> (1008)..(1008)
   <223> n is a, c, g, or t
<220>
   <221> misc_feature
   <222> (1015)..(1015)
   <223> n is a, c, g, or t
<220>
   <221> misc_feature
   <222> (1042)..(1042)
   <223> n is a, c, g, or t
<220>
   <221> misc_feature
   <222> (1048)..(1048)
   <223> n is a, c, g, or t
<400> 7
   tgtgagcgga tacattcccc tctagaaata attttgttta actttaagaa ggagatatnc      60
   atatgcacca tcatcatcat cattcttctg gtctggtgcc acgcggttct ggtatgaaag     120
   aaaccgctgc tgctaaattc gaacgccagc acatggacag cccagatctg ggtaccgatg     180
   acgacgacaa gatttcaggg tttgtgtttc gtcatgagat caaggacacc ttcctgagga     240
   cttacacgga cgctatgcag acttacaatg gcaatgatga gaggagccgg gcagtggacc     300
   atgtgcagcg cagcctgagc tgctgtggtg tgcagaacta caccaactgg agcaccagcc     360
   cctacttcct ggagcatggc atccccccca gctgctgcat gaacgaaact gattgtaatc     420
   cccaggatct acacaatctg actgtggccg ccaccaaagt taaccagaag ggttgttatg     480
   atctggtaac tagtttcatg gagactaaca tgggaatcat cgctggagac cgggcttctc     540
   ctcaaccatg gcgatatcgg atccgaattc tagctccgtc gacaagcttg cggccgcact     600
   cgagcaccac caccaccacc actgagatcc ggctgctaac aaagcccgaa aggaagctga     660
   gttggctgct gccaccgctg agcaataact agcataaccc cntggggcct ctaaacgggt     720
   cttgaggggt tttttgctga aaggaggaac tatatccgga ttggcgaatg ggacgcgccc     780
   tgtagcggcg cataaagcgc ggcgggtgtg gtggttacgc gcagcgtgac gnctaacttg     840
   ncagcgccct agcgcccnct cntttcgcnt ttcttccctt cctttctcgc ncgtttcgcc     900
   ggctttcccn gtcagctnta antcgggggg ctcccttagg gntccattta gtgctttacg     960
   gcccncaccc caaaaacttg attaaggtga ngggttcccg aatgggcntc ccccntgata     1020
   acggtttttc ccctttgacg tngagtcnct t                                   1051
<210> 8
   <211> 209
   <212> PRT
   <213> Homo sapiens
<220>
   <221> misc_feature
   <222> (188)..(188)
   <223> xaa can be any naturally occurring amino acid
<400> 8
   Met His His His His His His Ser Ser Gly Leu Val Pro Arg Gly Ser
   1               5                   10                  15
   Gly Met Lys Glu Thr Ala Ala Ala Lys Phe Glu Arg Gln His Met Asp
               20                  25                  30
   Ser Pro Asp Leu Gly Thr Asp Asp Asp Asp Lys Ile Ser Gly Phe Val
           35                  40                  45
   Phe Arg His Glu Ile Lys Asp Thr Phe Leu Arg Thr Tyr Thr Asp Ala
       50                  55                  60
   Met Gln Thr Tyr Asn Gly Asn Asp Glu Arg Ser Arg Ala Val Asp His
   65                  70                  75                  80
   Val Gln Arg Ser Leu Ser Cys Cys Gly Val Gln Asn Tyr Thr Asn Trp
                   85                  90                  95
   Ser Thr Ser Pro Tyr Phe Leu Glu His Gly Ile Pro Pro Ser Cys Cys
               100                 105                 110
   Met Asn Glu Thr Asp Cys Asn Pro Gln Asp Leu His Asn Leu Thr Val
           115                 120                 125
   Ala Ala Thr Lys Val Asn Gln Lys Gly Cys Tyr Asp Leu Val Thr Ser
       130                 135                 140
   Phe Met Glu Thr Asn Met Gly Ile Ile Ala Gly Asp Arg Ala Ser Pro
   145                 150                 155                 160
   Gln Pro Trp Arg Tyr Arg Ile Arg Ile Leu Ala Pro Ser Thr Ser Leu
                   165                 170                 175
   Arg Pro His Ser Ser Thr Thr Thr Thr Thr Thr Xaa Ile Arg Leu Leu
               180                 185                 190
   Thr Lys Pro Glu Arg Lys Leu Ser Trp Leu Leu Pro Pro Leu Ser Asn
           195                 200                 205
   Asn
<210> 9
   <211> 44
   <212> DNA
   <213> Homo sapiens
<400> 9
   gacgacgaca agatttcagg gtttgtgttt cgtcatgaga tcaa 44
<210> 10
   <211> 37
   <212> DNA
   <213> Homo sapiens
<400> 10
   gaggagaagc ccggtctcca gcgatgattc ccatgtt 37
<210> 11
   <211> 33
   <212> DNA
   <213> Homo sapiens
<400> 11
   gacgacgaca agatgcagcg cagcctgagc tgc 33
<210> 12
   <211> 33
   <212> DNA
   <213> Homo sapiens
<400> 12
   gacgacgaca agatcccccc cagctgctgc atg 33
<210> 13
   <211> 37
   <212> DNA
   <213> Homo sapiens
<400> 13
   gacgacgaca agatgaacga aactgattgt aatcccc 37
<210> 14
   <211> 30
   <212> DNA
   <213> Homo sapiens
<400> 14
   gacgacgaca agatggaccg ggcttctcct 30
<210> 15
   <211> 33
   <212> DNA
   <213> Homo sapiens
<400> 15
   gaggagaagc ccggtctagt tattgctcag egg 33

## Claims

1. An active polypeptide being a truncated form of SEQ ID NO:2 and consisting of the amino acid sequence of SEQ ID NO:3.

2. The polypeptide according to claim 1, wherein said polypeptide further comprises means for having it folded in an active three dimensional conformation wherein said means consists in any sequence comprising from 30 to 70 amino acids, preferably from 45 to 65 amino acids, more preferably from 50 to 60 amino acids, still more preferably of about 55 amino acids, said sequence being fused to the C-terminus of said polypeptide.

3. The polypeptide according to anyone of claims **1** to **2,** for inhibiting angiogenesis.

4. The polypeptide according to anyone of claims **1** to **2,** for treating tumors.

5. A medicament comprising a polypeptide according to anyone of claims 1 to 2.

6. A pharmaceutical composition comprising a polypeptide according to anyone of claims 1 to 2, and one or more pharmaceutically-acceptable excipients.

7. A pharmaceutical composition comprising a polypeptide according to anyone of claims 1 to 2, and one or more pharmaceutically-acceptable excipients, for treating cancer and/or tumours of the human or animal body.

8. A pharmaceutical composition according to claim **6 or 7,** further comprising at least one another active substance selected from anti-angiogenic substances or anti-tumour substances.

9. A pharmaceutical composition comprising synergistic effective amounts of
- a polypeptide according to anyone of claims 1 to 2, and
- a platinum complex selected from the group consisting of cisplatin and carboplatin.

10. The pharmaceutical composition according to anyone of claims **6-9,** being in a form suitable for topical, systemic, oral, subcutaneous, transderm, intramuscular or intraperitoneal administration.

11. The pharmaceutical composition according to anyone of claims **6-10,** wherein said polypeptide is present in an amount from 0.01 to 90% in weight, preferably from 0.1% to 10% in weight, more preferably from 1% to 5% in weight.

12. The medicament of claim **7,** or of the pharmaceutical composition of claims **6-11,** for use in the treatment of cancers and/or tumours.

13. The use according to claim **12,** wherein the tumours are solid tumours.

14. The use according to claim **12,** wherein the solid tumours are selected from sarcomas, carcinomas, and lymphomas.

## Patentansprüche

1. Aktives Polypeptid, welches eine gekürzte Form von SEQ ID NO: 2 ist und welches aus der Aminosäuresequenz von SEQ ID NO: 3 besteht.

2. Polypeptid gemäß Anspruch 1, wobei das Polypeptid ferner Mittel umfasst, um es in eine aktive dreidimensionale Konformation gefaltet zu haben, wobei das Mittel aus einer beliebigen Sequenz umfassend 30 bis 70 Aminosäuren besteht, vorzugsweise aus 45 bis 65 Aminosäuren, bevorzugterweise aus 50 bis 60 Aminosäuren, noch bevorzugterweise aus etwa 55 Aminosäuren, wobei die Sequenz an den C-Terminus des Polypeptids fusioniert ist.

3. Polypeptid gemäß einem beliebigen der Ansprüche 1 bis 2 zum Inhibieren von Gefäßwachstum.

4. Polypeptid gemäß einem beliebigen der Ansprüche 1 bis 2 zum Behandeln von Tumoren.

5. Medikament umfassend ein Polypeptid gemäß einem beliebigen der Ansprüche 1 bis 2.

6. Pharmazeutische Zusammensetzung umfassend ein Polypeptid gemäß einem beliebigen der Ansprüche 1 bis 2 und ein oder mehrere pharmazeutisch akzeptable Trägerstoffe.

7. Pharmazeutische Zusammensetzung umfassend ein Polypeptid gemäß einem beliebigen der Ansprüche 1 bis 2 und ein oder mehrere pharmazeutisch akzeptable Trägerstoffe zum Behandeln von Krebs und/oder Tumoren des menschlichen oder tierischen Körpers.

8. Pharmazeutische Zusammensetzung gemäß Anspruch 6 oder 7, ferner umfassend mindestens eine weitere aktive Substanz ausgewählt aus anti-angiogenetischen Substanzen oder Antitumorsubstanzen.

9. Pharmazeutische Zusammensetzung umfassend synergistisch wirksame Mengen
- eines Polypeptids gemäß einem beliebigen der Ansprüche 1 bis 2, und
- eines Platinumkomplexes ausgewählt aus der Gruppe bestehend aus Cisplatin und Carboplatin.

10. Pharmazeutische Zusammensetzung gemäß einem beliebigen der Ansprüche 6-9, welche in einer geeigneten Form zur topikalen, systemischen, oralen, subkutanen, transdermalen, intramuskulären oder intraperitonealen Verabreichung vorliegt.

11. Pharmazeutische Zusammensetzung gemäß einem beliebigen der Ansprüche 6-10, wobei das Polypeptid in einer Menge von 0,01 bis 90 Gew.-%, vorzugsweise von 0,1 bis 10 Gew.-%, noch bevorzugter von 1 bis 5 Gew.-% vorliegt.

12. Medikament gemäß Anspruch 7 oder pharmazeutische Zusammensetzung gemäß Ansprüchen 6-11 zur Verwendung in der Behandlung von Krebs und/oder Tumoren.

13. Verwendung gemäß Anspruch 12, wobei die Tumoren solide Tumoren sind.

14. Verwendung gemäß Anspruch 12, wobei die soliden Tumoren ausgewählt sind aus Sarkomen, Karzinomen und Lymphomen.

## Revendications

1. Un polypeptide actif étant une forme tronquée de SEQ ID NO : 2 et consistant en la séquence d'acides aminés SEQ ID NO : 3.

2. Le polypeptide selon la revendication **1,** ledit polypeptide comprenant en outre des moyens pour se replier dans une conformation tridimensionnelle active, lesdits moyens consistant en toute séquence comprenant de 30 à 70 acides aminés, préférablement de 45 à 65 acides aminés, plus préférablement de 50 à 60 acides aminés, encore plus préférablement d'environ 55 acides aminés, ladite séquence étant fusionnée au C-terminal dudit polypeptide.

3. Le polypeptide selon l'une quelconque des revendications **1** ou **2,** pour inhiber l'angiogénèse.

4. Le polypeptide selon l'une quelconque des revendications **1** ou **2,** pour traiter des tumeurs.

5. Un médicament comprenant un polypeptide selon l'une quelconque des revendications **1** ou **2.**

6. Une composition pharmaceutique comprenant un polypeptide selon l'une quelconque des revendications **1** ou **2,** et un ou plusieurs excipients pharmaceutiquement acceptables.

7. Une composition pharmaceutique comprenant un polypeptide selon l'une quelconque des revendications **1** ou **2,** et un ou plusieurs excipients pharmaceutiquement acceptables pour le traitement du cancer et/ou de tumeurs du corps humain ou animal.

8. Une composition pharmaceutique selon la revendication **6** ou **7,** comprenant en outre au moins une autre substance active sélectionnée parmi les substances anti-angiogéniques ou les substances anti-tumorales.

9. Une composition pharmaceutique comprenant des quantités effectives synergiques
- d'un polypeptide selon l'une quelconque des revendications **1** à **2,** et
- d'un complexe à base de platine sélectionné dans le groupe consistant en la cisplatine et la carboplatine.

10. La composition pharmaceutique selon l'une quelconque des revendications **6** à **9,** étant sous une forme acceptable pour une administration topique, systémique, orale, sous-cutanée, transdermique, intramusculaire, ou intra péritonéale.

11. La composition pharmaceutique selon l'une quelconque des revendications **6** à **10,** dans laquelle ledit polypeptide est présent dans une quantité de 0,01% à 90 % en poids, préférablement de 0,1% à 10 % en poids, plus préférablement de 1 % à 5% en poids.

12. Le médicament selon la revendication **7,** ou la composition pharmaceutique selon l'une quelconque des revendications **6** à **11,** pour son utilisation dans le traitement des cancers et/ou des tumeurs.

13. L'utilisation selon la revendication **12,** dans laquelle les tumeurs sont des tumeurs solides.

14. L'utilisation selon la revendication **12,** dans laquelle les tumeurs solides sont sélectionnées parmi les sarcomes, les carcinomes et les lymphomes.
